# EUROPÄISCHE PATENTSCHRIFT

(11) **EP 1 426 034 B1**
(45) Veröffentlichungstag und Bekanntmachung des Hinweises auf die Patenterteilung: **24.05.2006**
(21) Anmeldenummer: 04004305.1
(22) Anmeldetag: 26.08.1999
(51) Int. Cl.: A61K 8/41, A61K 8/49, A61K 8/69, A61Q 7/00

(54) **Haarwuchsmittel**
Hair growth tonic
Produit stimulant la croissance des cheveux

(30) Priorität: 01.10.1998 DE 19845202
(43) Veröffentlichungstag der Anmeldung: 09.06.2004
(62) Teilanmeldung aus: 99970016.4
(73) Patentinhaber: Wella Aktiengesellschaft, 64274 Darmstadt (DE)
(72) Erfinder: Damm, Peter, 58540 Meinerzhagen (DE); Noser, Friedrich, Dr., 1729 Bonnefontaine (CH); Schweickert, Rainer, 64347 Griesheim (DE); Heinrich, Hans, 68519 Viernheim (DE); Reinemund, Eva, 65558 Hirschberg (DE)

(56) Entgegenhaltungen:
- EP-A- 0 867 171
- WO-A-97/32562
- WO-A-98/22078
- WO-A-98/23247
- US-A- 5 059 606

## Beschreibung

Die vorliegende Erfindung betrifft die Verwendung bestimmter cyclischer Verbindungen zur Förderung des Haarwuchses und/oder Verringerung des Haarausfalls sowie ein Verfahren zur Verringerung des Haarausfalls und zur Förderung des Haarwachstums.

Die Kopfhaut des Menschen beherbergt normalerweise 100 000 bis 150 000 Haarfollikel beziehungsweise Haare. Die Haarfollikel beziehungsweise Haarwurzeln oder Haarbulbi sind die haarbildenden Organe. Die langen, starken Haare, welche das Kopfhaarkleid aufbauen, werden Terminalhaare genannt. Die sehr feinen, sehr kurzen, knapp bis über die Hautoberfläche ragenden, sich beispielsweise an den Rändern des Kopfhaarkleides oder auf der Stirn befindenden Haare werden als Flaumhaare beziehungsweise Vellushaare bezeichnet. Das Wachstum der Haare verläuft nicht in kontinuierlicher, sondern in cyclischer Form. Drei Wachstumsphasen werden unterschieden: (1) Das Anagen, während dem das Haar wächst. (2) Das Katagen, während dem sich der Haarfollikel auf die nächste Phase -das Telogen- vorbereitet und das bisher wachsende Haar in ein Ruhehaar beziehungsweise Kolbenhaar umgewandelt wird. (3) Das Telogen ist die Ruhephase, während der das Haarwachstum völlig einhestellt wird. Auf ein gegenwärtig nicht identifiziertes Signal hin erwacht der Telogenfollikel zu neuer Aktivität beziehungsweise beginnt ein neues Anagen, in dessen Verlauf ein neues Haar wächst und das vorhandene Kolbenhaar abgestoßen wird. Dieser Haarwuchszyklus läuft das ganze Leben hindurch unverändert weiter, und zwar gleicherweise sowohl im Falle der langen, starken Terminalhaare als auch der sehr kurzen Flaumhaare beziehungsweise Vellushaare. Voraussetzung für ein normales, gesundes Wachstum des Kopfhaares ist ein perfekt funktionerender Organismus und dessen optimale Versorgung mit allen notwendigen Nähr- und Hilfsstoffen. Vielerlei Faktoren können das Wachstum des Kopfhaares beeinträchtigen, nämlich Fehlernährung, Mangelernährung, schwere Krankheiten, Medikamente, psychischer Stress und vorübergehende Störungen des Hormonhaushaltes im Organismus. Ein durch derartige Faktoren bewirkter Haarausfall ist in der Regel vorübergehend.

Welche Form des Haares beziehungsweise Terminalhaar oder Flaumhaar, oder auch Haarformen, welche zwischen Terminalhaar und Flaumhaar liegen, die Haarfollikel der Kopfhaut im Verlauf des Lebens bilden, hängt in starkem Maße von der den Haarfollikeln innewohnenden genetischen Disposition beziehungsweise Erbanlage sowie den männlichen Sexualhormonen ab. Bei Personen mit der Erbanlage zu männlichem beziehungsweise androgenem Haarausfall beginnen sich im Verlauf der Pubertät auf der Kopfhaut die Terminalhaarfollikel in Vellushaarfollikel umzuwandeln. Dieser Umwandlungsprozeß kann sehr unterschiedlich weit fortschreiten, wobei Kopfhautbezirke entstehen, welche teilweise, überwiegend oder vollständig mit Flaumhaarfollikeln, beziehungsweise Flaumhaaren besetzt sind beziehungsweise die androgene Kahlheit oder Glatze vom männlichen Typ (männliche Glatze) aufweisen. Der männliche Haarausfall ist eine irreversible Form des Haarausfalles, es sei denn, es ließen sich Mittel finden, welche die Transformation von Terminalhaarfollikeln in Flaumhaarfollikel verlangsamen, anhalten oder umkehren könnten, beziehungsweise im letzteren Falle Flaumhaarfollikel wieder in Terminalhaarfollikel rücktransformieren könnten.

In der Literatur sind wiederholt Stoffe, wie zum Beispiel Minoxidil, der 5-alpha-Reduktase-Hemmstoff Finasterid (Propecia), beschrieben worden, welche die Umwandlung von Terminalhaarfollikeln in Flaumhaarfollikel verzögern beziehungsweise in begrenztem Masse stoppen oder auch umkehren sollen. Zwar fördert Minoxidil, ein Antihypertensivum, bei peroraler Verabreichung auf dem Großteil der Körperhaut in sehr ausgeprägtem Masse die Umwandlung von Flaumhaarfollikeln in Terminalhaarfollikel, auf der Kopfhaut indessen ist die entsprechende Wirkung des Minoxidils nur sehr schwach und unter kosmetischem Gesichtspunkten von sehr begrenztem Ausmaß, wobei eine epikutane Anwendung des Minoxidils auf der Kopfhaut diesen Sachverhalt praktisch nicht verändert. Finasterid, vermag zwar bei peroraler Gabe die Transformation von Terminalhaarfollikeln in Vellushaarfollikel in begrenztem Ausmaß zu bremsen, anzuhalten oder umzukehren, dieser Effekt ist jedoch unter kosmetischen Gesichtspunkten ebenfalls nicht in jeder Hinsicht befriedigend.

Da weit mehr als die Hälfte der Männer und in zunehmendem Maße auch Frauen vom Haarausfall betroffen sind, besteht eine starke Nachfrage nach Mitteln, welche den Haarausfall, und insbesondere den männlichen Haarausfall, in all seinen Erscheinungsformen bremsen, anhalten oder gar rückgängig machen können.

Überraschenderweise wurde nunmehr gefunden, daß bestimmte heteroaromatische Verbindungen der Formel (I) oder (II) alleine oder in Kombination miteinander, epikutan auf die Kopfhaut appliziert insbesondere bei männlichem Haarausfall das Bild des Haarausfalls entscheidend verbessern können, wobei sowohl eine Verlangsamung oder ein Anhalten der Umwandlung von Terminalhaarfollikeln in Vellushaarfollikel, als auch eine Umkehrung der Umwandlung von Terminalhaarfollikeln in Vellushaarfollikel erfolgt.

Gegenstand der vorliegenden Erfindung ist daher die Verwendung einer oder mehrerer der Verbindungen der Formel (I) und/oder (II) oder deren physiologisch verträglichen Salzen zur Förderung des Haarwuchses und/oder Verringerung des Haarausfalls, 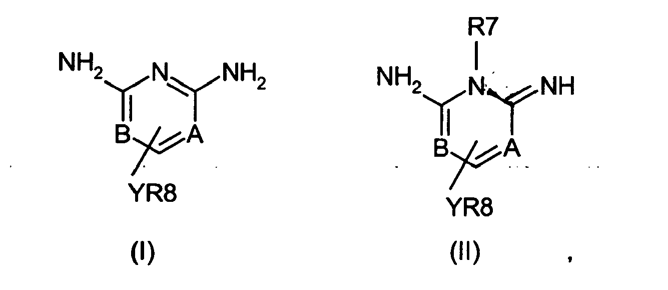 wobei die Restgruppen **A, B, Y, R7** und **R8** unabhängig voneinander die folgende Bedeutung haben:
**A** und **B** stehen unabhängig voneinander für eine CH-Gruppe oder ein Stickstoffatom, wobei jedoch mindestens eine der beiden Gruppen **A** und
**B** eine CH-Gruppe darstellt;
**Y** steht für -O-, -(CH₂)ₙ-, -NH-CH₂-, -CH₂-NH-,N=N-, -CH=CH-, -CH₂-O-, -O-CH₂-, -N=CH-, -CH=N-, -(CH₂-CH₂-O)ₘ- oder -(O-CH₂-CH₂)ₘ-, mit m und n gleich einer ganzen Zahl von 1 bis 4;
**R7** ist gleich OH, -OSO₃H, -O-(C₁- bis C₄-Alkyl) oder -OCOCH₃;
**R8** ist gleich einer C₁- bis C₆-Alkylgruppe, einer C₁- bis C₆-Hydroxyalkylgruppe, einer C₂- bis C₆-Hydroxyalkenylgruppe, einer Arylgruppe, einer Heteroarylgruppe oder einer C₂- bis C₆-Alkenylcarbonsäure- gruppe.

Bevorzugt sind Verbindungen der Formel (I) mit A gleich -CH- und **B** gleich -CH- oder -N-; **Y** gleich -N=N-,-(CH₂)ₙ-, -CH=CH-, -CH=N- oder - N=CH- und **R8** gleich einer C₁- bis C₆-Alkylgruppe, einer C₁- bis C₆₋Hydroxyalkylgruppe, einer Phenylgruppe, einer Benzylgruppe oder einer Pyridylgruppe; wobei Verbindungen der Formel (II) mit **A** und **B** gleich -CH- ; **Y** gleich -N=N- und **R8** gleich einer Pyridylgruppe besonders bevorzugt sind.

Ebenfalls bevorzugt sind Verbindungen der Formel (II) mit **A** gleich -CH- und **B** gleich -CH- oder -N-; **Y** gleich -N=N-,-(CH₂)ₙ-, -CH=CH-, -CH=N- oder -N=CH-; **R7** gleich OH, -OSO₃H oder -O-(C₁- bis C₄-Alkyl) und **R8** gleich einer C₁- bis C₆-Alkylgruppe, einer C₁- bis C₆-Hydroxyalkylgruppe, einer Phenylgruppe, einer Benzylgruppe oder einer Pyridylgruppe; wobei Verbindungen der Formel (II) mit **A** und **B** gleich -CH-; **Y** gleich -N=N; **R7** gleich -OH (einschließlich des entsprechenden tautomeren N-Oxides) und **R8** gleich einer Pyridylgruppe besonders bevorzugt sind.

Besonders bevorzugt sind hierbei solche Mittel, welche eine oder mehrerer der folgenden Verbindungen enthalten: 2,6-Diamino-3-((pyridin-3-yl)-azo)-pyridin oder 2,6-Diamino-3-((pyridin-3-yl)-azo)-pyridin-1-oxid. Unter diesen Verbindungen ist insbesondere das 2,6-Diamino-3-((pyridin-3-yl)-azo)-pyridin zu nennen.

Die Verwendung erfolgt in Form eines mindestens eine Verbindung der Formel (I) oder (II) enthaltenden kosmetischen Mittels, welches in jeder für die Applikation auf die Haare und die Kopfhaut geeigneten Form vorliegen kann, insbesondere in Form einer wäßrigen, alkoholischen oder wäßrigalkoholischen Zubereitung, beispielsweise als Lösung, Gel, Creme, Emulsion oder Dispersion. Ebenfalls ist es möglich, diese Mittel im Gemisch mit üblichen unter Druck verflüssigten Treibmitteln, beispielsweise Chlorfluoralkanen, wie zum Beispiel CCl₃F, CCl₂F₂, C₂Cl₃F₃, CCl₂F₂, CHCl₂F und (CClF₂)₂, leichtflüchtigen Kohlenwasserstoffen, wie zum Beispiel n-Butan und n-Propan, oder auch Dimethylether, Kohlendioxid, Distickstoffmonoxid, Stickstoff, Methylenchlorid und 1,1,1-Trichlorethan, aus einem Druckbehälter als Spray oder Schaum zu entnehmen.

Die Zubereitungsformen des erfindungsgemäßen kosmetischen Mittels können insbesondere solche sein, die längere Zeit auf dem Haar und der Kopfhaut verbleiben, wie zum Beispiel Einlegemittel, Frisiergele, Haarpomaden, Haarkuren, Haaröle und Haarwasser. Besonders bevorzugt sind hierbei Haarwasser sowie sogenannte "Retardpräparate", welche den Wirkstoff über einen längeren Zeitraum gleichmäßig abgeben, oder die Konfektionierung der Verbindungen der Formel (I) und (II) als Liposome .

Der Gesamtgehalt an Verbindungen der Formel (I) und/oder (II) beträgt in dem erfindungsgemäßen Mittel vorzugsweise etwa 0,001 bis 10 Gewichtsprozent, wobei eine Menge von 0,05 bis 3 Gewichtsprozent, besonders bevorzugt ist.

Die Zusammensetzung des erfindungsgemäßen kosmetischen Mittels stellt eine Mischung der Verbindungen der Formel (I) und/oder (II) mit den für solche Mittel üblichen Bestandteilen, wie Trägerstoffen und Zusatzstoffen, dar.

Als Trägerstoffe kommen im allgemeinen solche in Betracht, welche die perkutane Resorption der Wirkstoffe erhöhen, die Wirkstoffkomponenten nicht nachteilig beeinflussen und gleichzeitig unschädlich gegenüber der menschlichen Haut sind. Solche Trägerstoffe sind beispielsweise Wasser, niedere aliphatische Alkohole, wie zum Beispiel Ethanol, Propanol und Isopropanol, sowie Mischungen dieser Stoffe. Vorteilhaft sind aber auch Mischungen der vorgenannten Verbindungen mit 1 bis 30 Gewichtsprozent 1,2-Propandiol.

Als übliche Zusatzstoffe in den erfindungsgemäßen kosmetischen Mitteln kommen zum Beispiel in Betracht: Carrierverbindungen oder Penetrationsbeschleuniger, wie zum Beispiel Benzylalkohol, 2-Benzyloxyethanol, α-Hydroxycarbonsäureester, Vanillin, p-Hydroxyanisol, 3-Hydroxy-4-methoxy-benzahldehyd, 2-Phenoxyethanol, Salicylaldehyd, 3,5-Dihydroxy-benzaldehyd, 3,4-Dihydroxybenzaldehyd, 4-Hydroxyphenylacetamid, p-Hydroxybenzoesäuremethylester, p-Hydroxybenzaldehyd, m-Kresol, Hydrochinonmono-methylether, o-Fluorphhenol, m-Fluorphenol, p-Fluor-phenol, 2-(2'-Hydroxyphenoxy)-ethanol, 3,4-Methylen-dioxy-phenol, Resorcinmonomethylether, 3,4-Dimethoxyphenol, 3-Trifluormethyl-phenol, Resorcinmonoacetat, Ethylvanillin, 2-Thiophenethanol , Milchsäurebutylester und Glykolsäurebutylester; Verdickungsmittel, wie beispielsweise Kaolin, Bentonit, Fettsäuren, höhere Fettalkohole, Stärke, Polyacrylsäure, Cellulosederivate, Alginate, Vaseline oder Paraffinöl; Verbindungen aus den Klassen der anionischen, kationischen amphoteren, zwitterionischen oder nichtionischen Tenside oder oberflächenaktiven Agenzien, wie zum Beispiel Fettalkoholsulfate, Fettalkoholethersulfate, Alkylsulfonate, Alkylbenzolsulfate, quartäre Ammoniumsalze, Alkylbetaine und oxethylierte Fettsäureester; ferner Trübungsmittel, wie zum Beispiel Polyethylenglykolester; Schaumstabilisatoren, wie beispielsweise Fettamide; Schaumsynergisten; Sequestriermittel; Pufferstoffe; Konservierungsmittel; Lösungsvermittler; Parfümöle; natürliche oder synthetische kosmetische Polymere, wie beispielsweise Cellulosederivate, Schellack, Pektine, Polyvinylpyrrolidon, Polyvinylacetat, Polyacrylverbindungen, wie zum Beispiel Acrylsäure- oder Methacrylsäurepolymerisate, basische Polymerisate von Estern aus Acrylsäure oder Methacrylsäure mit Aminoalkoholen, Polyacrylnitril und Chitosanderivate; Haarkonditionierungsmittel; Wirkstoffe gegen Kopfschuppen; Pflanzenextrakte sowie haarpflegende Bestandteile, wie zum Beispiel Proteinhydrolysate, Lanolinderivate, Cholesterin, Pantothensäure oder Betain.

Selbstverständlich können die erfindungsgemäßen Mittel zusätzlich bekannte, das Wachstum des Haares gesund erhaltende und/oder die Gesundung des Haarwachstums bei Störungen physiologischer und nichtphysiologischer Art unterstützende beziehungsweise fördernde Wirkstoffe, wie zum Beispiel Minoxidil, Diazoxyd, Cyclosporin A, Diphenylhydantoin, Acetazolamid, Antiandrogene steroidaler und nicht-steroidaler Art wie Cyproteronacetat, Oxendolon, Spironolacton, 5-alpha-Reduktasehemmstoffe, ausgewählte Extrakte natürlichen Ursprungs, Retinoide, Östrogene, Vitamine wie zum Beispiel Biotin, Spurenelemente, Neuropeptide, Nährstoffe insbesondere des essentiellen Typs, Cytokine, Neurotrophine, Neurotrophinrezeptor-Antagonisten, antimicrobielle Substanzen, steroidale oder nicht-steroidale entzündungshemmende Substanzen, Calciumantagonisten oder Kaliumkanal-Öffner (Kaliumkanal-Agonisten), enthalten.

Die vorliegende Erfindung betrifft weiterhin ein Haarbehandlungsverfahren zur Verringerung des Haarausfalls und zur Förderung des Haarwachstums, welches dadurch gekennzeichnet ist, daß man eine ausreichende Menge des zuvor beschriebenen, mindestens eine Verbindung der Formel (I) oder (II) enthaltenden erfindungsgemäßen Mittels, im allgemeinen etwa 1 bis 30 Milliliter (vorzugsweise etwa 2 bis 15 Milliliter), auf die Haare und die Kopfhaut aufbringt, anschließend die Kopfhaut (vorzugsweise etwa 1 bis 5 Minuten lang) massiert und das Mittel auf den Haaren und der Kopfhaut für einen längeren Zeitraum, vorzugsweise mindestens 24 Stunden, beläßt.

Die Behandlung wird vorzugsweise 1 bis 2mal täglich und während einer Zeit von 3 bis 24 Monaten vorgenommen. Der Abstand zwischen den Anwendungen kann dann gegebenenfalls vergrößert werden.

Die folgende Beispiele sollen den Gegenstand der Erfindung näher erläutern, ohne diesen hierauf zu beschränken.

### Beispiele

### Beispiel 1: Haarwasser

| | |
|---|---|
| 1,0 g | 1-[N-(2' -Nitro-4' -trifluoromethyl-phenyl)-amino]-propan-2,3-diol |
| 1,0 g | 2,6-Diamino-3-((pyridin-3-yl)-azo)-pyridin |
| 3,0 g | 1,2-Propandiol |
| 0,3 g | Parfümöl |
| ad 100,0 g | Ethanol (96-prozentig) |

Jeweils 15 Milliliter des obigen Haarwassers werden einmal täglich (alle 24 Stunden) auf die Haare und die Kopfhaut aufgetragen und 1 Minute lang in die Kopfhaut einmassiert.

### Beispiel 2: Haarwasser

| | |
|---|---|
| 2,0 g | 1-[N-(2'-Nitro-4'-trifluoromethyl-phenyl)-amino]-propan-2,3-diol |
| 1,0 g | 2,6-Diamino-3-((pyridin-3-yl)-azo)-pyridin |
| 0,1 g | Menthol |
| 0,3 g | Parfümöl |
| ad 100,0 g | Ethanol (96-prozentig) |

Das obige Haarwasser wird zweimal täglich auf die Haare und die Kopfhaut aufgetragen und 2 Minute lang in die Kopfhaut einmassiert.

### Beispiel 3: Haarwasser

| | |
|---|---|
| 1,0 g | 1 -[N-(2'-Nitro-4'-trifluoromethyl-phenyl)-amino]-propan-2,3-diol |
| 0,5 g | 2,6-Diamino-3-((pyridin-3-yl)-azo)-pyridin |
| 0,2 g | hydriertes Rizinusöl mit 60 mol EO (Cremophor RH 60 der BASF AG/Ludwigshafen) |
| 0,3 g | Parfümöl |
| 40,0 g | Ethanol (96-prozentig) |
| ad 100,0 g | Wasser |

Das obige Haarwasser (jeweils 20 Milliliter) wird einmal täglich auf die Haare und die Kopfhaut aufgetragen und 1 bis 2 Minuten lang in die Kopfhaut einmassiert.

### Beispiel 4: Haarwasser

| | |
|---|---|
| 1,0 g | 1-[N-(2'-Nitro-4'-trifluoromethyl-phenyl)-amino]-3-chlor-2-hydroxy-propan |
| 1,0 g | 2,6-Diamino-3-((pyridin-3-yl)-azo)-pyridin |
| 3,0 g | 1,2-Propandiol |
| 0,3 g | Parfümöl |
| ad 100,0 g | Ethanol (96-prozentig) |

Jeweils 15 Milliliter des obigen Haarwassers werden einmal täglich (alle 24 Stunden) auf die Haare und die Kopfhaut aufgetragen und 1 Minute lang in die Kopfhaut einmassiert.

### Beispiel 5: Haarwasser

| | |
|---|---|
| 2,0 g | 1 -[N-(2'-Nitro-4'-trifluoromethyl-phenyl)-amino]-2-hydroxy-2-trifluoromethylethan |
| 1,0 g | 2,6-Diamino-3-((pyridin-3-yl)-azo)-pyridin |
| 0,1 g | Menthol |
| 0,3 g | Parfümöl |
| ad 100,0 g | Ethanol (96-prozentig) |

Das obige Haarwasser wird zweimal täglich auf die Haare und die Kopfhaut aufgetragen und 2 Minute lang in die Kopfhaut einmassiert.

### Beispiel 6: Haarwasser

| | |
|---|---|
| 0,7 g | 1-[N-(2'-Nitro-4'-trifluoromethyl-phenyl)-amino]-propan-2,3-diol |
| 0,9 g | 2,6-Diamino-3-((pyridin-3-yl)azo)-pyridin-1-oxid |
| 0,2 g | hydriertes Rizinusöl mit 60 mol EO (Cremophor RH 60 der BASF AG/Ludwigshafen) |
| 0,3 g | Parfümöl |
| 50,0 g | Ethanol (96-prozentig) |
| ad 100,0 g | Wasser |

Das obige Haarwasser (jeweils 20 Milliliter) wird einmal täglich auf die Haare und die Kopfhaut aufgetragen und 1 bis 2 Minuten lang in die Kopfhaut einmassiert.

### Beispiel 7: Haarkur

| | |
|---|---|
| 0,30 g | 1-[N-(2'-Nitro-4'-trifluoromethyl-phenyl)-amino]-propan-2,3-diol |
| 0,01 g | 2,6-Diamino-3-((pyridin-3-yl)-azo)-pyridin-1-oxid |
| 0,20 g | hydriertes Rizinusöl mit 60 mol EO (Cremophor RH 60 der BASF AG/Ludwigshafen) |
| 0,30 g | Parfümöl |
| 0,50 g | Cetylalkohol |
| ad 100,00 g | Wasser |

Die obige Haarkur (jeweils 15 Milliliter) wird einmal täglich auf die Haare und die Kopfhaut aufgetragen und 2 Minuten lang in die Kopfhaut einmassiert. Nach einer Einwirkungszeit von ca. 24 Stunden wird die Haarkur mit lauwarmem Wasser ausgespült.

### Beispiel 8: Emulsionsförmiqes Haarwuchsmittel

| | |
|---|---|
| 0,4 g | 1-[N-(2'-Nitro-4'-trifluoromethyl-phenyl)-amino)-propan-2,3-diol |
| 0,1 g | 2,6-Diamino-3-((pyridin-3-yl)-azorpyridin |
| 30,0 g | teilweise gehärtetes Erdnußöl (oleum arachidis hydrogenatum) |
| 5,0 g | Cetylstearylalkohol |
| 0,2 g | Parfümöl |
| 10,0 g | Propylenglykol |
| ad 100,0 g | Wasser |

Das obige Haarwuchsmittel (jeweils 20 Milliliter) wird einmal täglich auf die Haare und die Kopfhaut aufgetragen und 1 bis 2 Minuten lang in die Kopfhaut einmassiert. Nach einer Einwirkungszeit von ca. 24 Stunden wird das Mittel mit lauwarmem Wasser ausgespült.

Alle Gewichtsangaben stellen, soweit nicht anders angegeben, Gewichtsprozente dar.

## Patentansprüche

1. Verwendung einer oder mehrerer der Verbindungen der Formel (I) und/oder (II) oder deren physiologisch verträglichen Salzen zur Förderung des Haarwuchses und/oder Verringerung des androgenen Haarausfalls, 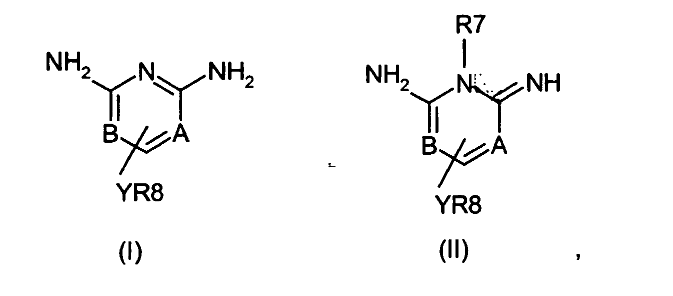 wobei die Restgruppen **A, B, Y, R7** und **R8** unabhängig voneinander die folgende Bedeutung haben:
**A** und **B** stehen unabhängig voneinander für eine CH-Gruppe oder ein Stickstoffatom, wobei jedoch mindestens eine der beiden Gruppen **A** und
**B** eine CH-Gruppe darstellt;
**Y** steht für -O-, -(CH₂)ₙ-, -NH-CH₂-, -CH₂-NH-, -N=N-, -CH=CH-, -CH₂-O-, -O-CH₂-, -N=CH-, -CH=N-, -(CH₂-CH₂-O)ₘ- oder -(O-CH₂-CH₂)ₘ-, mit m und n gleich einer ganzen Zahl von 1 bis 4;
**R7** ist gleich OH, -OSO₃H, -O-(C₁- bis C₄-Alkyl) oder -OCOCH₃;
**R8** ist gleich einer C₁- bis C₆-Alkylgruppe, einer C₁- bis C₆₋Hydroxyalkylgruppe, einer C₂- bis C₆-Hydroxyalkenylgruppe, einer Arylgruppe, einer Heteroarylgruppe oder einer C₂- bis C₆-Alkenylcarbonsäuregruppe.

2. Verwendung nach Anspruch 1, **dadurch gekennzeichnet, dass** die Verbindung der Formel (I) ausgewählt ist aus Verbindungen der Formel (I) mit **A** gleich -CH- und **B** gleich -CH- oder -N-; **Y** gleich -N=N-,-(CH₂)ₙ-, - CH=CH-, -CH=N- oder -N=CH- und **R8** gleich einer C₁- bis C₆₋Alkylgruppe, einer C₁- bis C₆-Hydroxyalkylgruppe, einer Phenylgruppe, einer Benzylgruppe oder einer Pyridylgruppe.

3. Verwendung nach Anspruch 1, **dadurch gekennzeichnet, dass** die Verbindung der Formel (II) ausgewählt ist aus Verbindungen der Formel (II) mit **A** gleich -CH- und **B** gleich -CH- oder -N-; **Y** gleich -N=N-,-(CH₂)ₙ-, - CH=CH-, -CH=N- oder -N=CH-; **R7** gleich OH, -OSO₃H oder -O-(C₁- bis C₄-Alkyl) und **R8** gleich einer C₁- bis C₆-Alkylgruppe, einer C₁- bis C₆₋Hydroxyalkylgruppe, einer Phenylgruppe, einer Benzylgruppe oder einer Pyridylgruppe.

4. Verwendung nach einem der Ansprüche 1 bis 3, **dadurch gekennzeichnet, daß** die Verbindung der Formel (I) und/oder (II) ausgewählt ist aus 2,6-Diamino-3-((pyridin-3-yl)-azo)-pyridin und 2,6-Diamino-3-((pyridin-3-yl)-azo)-pyridin-1-oxid oder Kombinationen dieser Verbindungen.

5. Verwendung nach Anspruch 4, **dadurch gekennzeichnet, daß** die Verbindung der Formel (I) und/oder (II) das 2,6-Diamino-3-((pyridin-3-yl)-azo)-pyridin ist.

6. Verwendung nach einem der Ansprüche 1 bis 4, **dadurch gekennzeichnet, daß** die Verwendung in Form eines Retardpräparates oder eines Haarwassers erfolgt.

7. Verfahren zur Verringerung des Haarausfalls und zur Förderung des Haarwachstums, **dadurch gekennzeichnet, daß** man eine ausreichende Menge eines mindestens eine Verbindung der Formel (I) und/oder (II) gemäß einem der Ansprüche 1 bis 5 oder deren physiologisch verträgliches Salz enthaltenden Mittels auf die Kopfhaut und die Haare aufträgt, anschließend die Kopfhaut massiert und das Mittel für mindestens 24 Stunden auf der Kopfhaut beläßt.

## Claims

1. Use of one or more of the compounds of the formula (I) and/or (II) or physiologically compatible salts thereof for promoting hair growth and/or reducing androgenic hair loss, 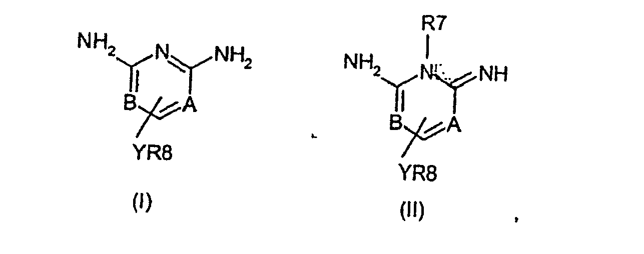 where the radical groups **A, B, Y, R7** and **R8,** independently of one another, have the following meanings:
**A** and **B,** independently of one another, are a CH group or a nitrogen atom, but where at least one of the two groups **A** and B is a CH group;
**Y** is -O-, - (CH₂) -, -NH-CH₂-, -CH₂-NH-, -N=N-, -CH=CH-, -CH₂-O-, -O-CH₂-, -N=CH-, -CH=N-, - (CH₂-CH₂-O) ₘ- or -(O-CH₂CH₂)ₘ-, where m and n are an integer from 1 to 4;
**R7** is OH, -OSO₃H, -O-(C₁- to C₄-alkyl) or -OCOCH₃;
**R8** is a C₁- to C₆-alkyl group, a C₁- to C₆-hydroxyalkyl group, a C₂-C₆-hydroxyalkenyl group, an aryl group, a heteroaryl group or a C₂- to C₆-alkenyl carboxylic acid group.

2. Use according to Claim 1, **characterized in that** the compound of the formula (I) is chosen from compounds of the formula (I) where **A** is -CH- and **B** is -CH- or -N-; **Y** is -N=N-, -(CH₂)ₙ-, -CH=CH-, -CH=N- or -N=CH- and **R8** is a C₁- to C₆-alkyl group, a C₁- to C₆₋hydroxyalkyl group, a phenyl group, a benzyl group or a pyridyl group.

3. Use according to Claim 1, **characterized in that** the compound of the formula (II) is chosen from compounds of the formula (II) where **A** is -CH- and **B** is -CH- or -N-; **Y** is -N=N-, - (CH₂) ₙ-, -CH=CH-, -CH=N- or -N=CH-; **R7** is OH, -OSO₃H or -O-(C₁- to C₄-alkyl) and **R8** is a C₁- to C₆-alkyl group, a C₁- to C₆-hydroxyalkyl group, a phenyl group, a benzyl group or a pyridyl group.

4. Use according to one of Claims 1 to 3, **characterized in that** the compound of the formula (I) and/or (II) is chosen from 2,6-diamino-3-((pyridin-3-yl)azo)pyridine and 2,6-diamino-3-((pyridin-3-yl)azo)pyridine 1-oxide or combinations of these compounds.

5. Use according to Claim 4, **characterized in that** the compound of the formula (I) and/or (II) is 2,6-diamino-3-((pyridin-3-yl)azo)pyridine.

6. Use according to one of Claims 1 to 4, **characterized in that** the use takes place in the form of a slow-release preparation or a hair tonic.

7. Method of reducing hair loss and of promoting hair growth, **characterized in that** a sufficient amount of an agent comprising at least one compound of the formula (I) and/or (II) according to one of Claims 1 to 5 or physiologically compatible salt thereof is applied to the scalp and the hair, then the scalp is massaged and the agent is left on the scalp for at least 24 hours.

## Revendications

1. Utilisation d'un ou plusieurs des composés de formule (I) et/ou de formule (II) ou de leurs sels physiologiquement acceptables, pour favoriser la pousse des cheveux et/ou réduire l'alopécie androgène, 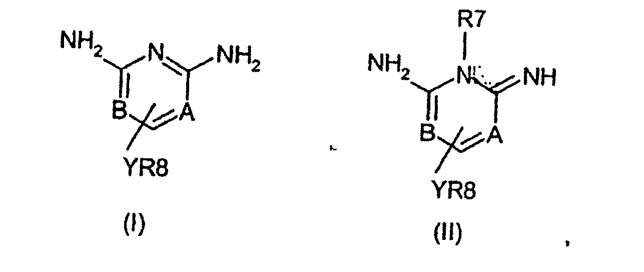 formules dans lesquelles les radicaux **A, B, Y, R7** et **R8** ont, indépendamment les uns des autres, les significations suivantes :
**A** et **B** représentent, indépendamment l'un de l'autre, un groupe CH ou un atome d'azote, au moins l'un des deux groupes **A** et **B** représentant toutefois un groupe CH ;
**B** représente un groupe CH ;
**Y** représente -O-, -(CH₂)ₙ-, -NH-CH₂-, -CH₂-NH-, -N=N-, -CH=CH-, -CH₂O-, -O-CH₂-, -N=CH-, -CH=N-, -(CH₂-CH₂-O)ₘ- ou (O-CH₂-CH₂) ₘ-, m et n représentant un nombre entier allant de 1 à 4 ;
**R7** représente OH, -OSO₃H, -O-(alkyle en C₁-C₄) ou -OCOCH₃ ;
**R8** représente un groupe alkyle en C₁-C₆, un groupe hydroxyalkyle en C₁-C₆, un groupe hydroxyalcényle en C₂-C₆, un groupe aryle, un groupe hétéroaryle ou un groupe alcényl (C₂-C₆) -carboxy.

2. Utilisation selon la revendication 1, **caractérisée en ce que** le composé de formule (I) est choisi parmi les composés de formule (I) dans lesquels **A** représente -CH- et **B** représente -CH- ou -N- ; **Y** représente -N=N-, -(CH₂)ₙ-, -CH=CH-, -CH=N- ou -N=CH- et **R8** représente un groupe alkyle en C₁-C₆, un groupe hydroxyalkyle en C₁-C₆, un groupe phényle, un groupe benzyle ou un groupe pyridyle.

3. Utilisation selon la revendication 1, **caractérisée en ce que** le composé de formule (II) est choisi parmi les composés de formule (II) dans lesquels **A** représente -CH- et **B** représente -CH- ou -N- ; **Y** représente -N=N-, -(CH₂)ₙ-, -CH=CH-, -CH=N- ou -N=_{CH}- ; **R7** représente OH, -OSO₃H ou -O-(alkyle en C₁-C₄) et **R8** représente un groupe alkyle en C₁-C₆, un groupe hydroxyalkyle en C₁-C₆, un groupe phényle, un groupe benzyle ou un groupe pyridyle.

4. Utilisation selon l'une quelconque des revendications 1 à 3, **caractérisée en ce que** le composé de formule (I) et/ou de formule (II) est choisi parmi la 2,6-diamino-3-((pyridin-3-yl)-azo)-pyridine et le 2,6-diamino-3-((pyridin-3-yl)-azo)-pyridine-1-oxyde ou des associations de ces composés.

5. Utilisation selon la revendication 4, **caractérisée en ce que** le composé de formule (I) et/ou de formule (II) est la 2,6-diamino-3-((pyridin-3-yl)-azo)-pyridine.

6. Utilisation selon l'une quelconque des revendications 1 à 4, **caractérisée en ce que** l'utilisation s'effectue sous forme d'une préparation retard ou d'une lotion capillaire.

7. Procédé pour diminuer la chute des cheveux et pour favoriser la pousse des cheveux, **caractérisé en ce qu'**on applique sur le cuir chevelu et les cheveux une quantité suffisante d'une composition contenant au moins un composé de formule (I) et/ou de formule (II) selon l'une quelconque des revendications 1 à 5 ou un sel physiologiquement acceptable d'un tel composé, on masse ensuite le cuir chevelu et on laisse la composition pendant au moins 24 heures sur le cuir chevelu.
